# EUROPÄISCHE PATENTANMELDUNG

(11) **EP 0 787 486 A1**
(43) Veröffentlichungstag der Anmeldung: **06.08.1997**
(21) Anmeldenummer: 97101508.6
(22) Anmeldetag: 31.01.1997
(51) Int. Cl.: A61K 7/50, A61K 7/48

(54) **Wässerige Zubereitungen, enhaltend Alkylpolyglykoside und ein Polymer**

(30) Priorität: 08.02.1996 DE 19604466
(71) Anmelder: BASF AKTIENGESELLSCHAFT, 67056 Ludwigshafen (DE)
(72) Erfinder: Oppenländer, Knut, Dr., 67061 Ludwigshafen (DE); Oetter, Günter, Dr., 67227 Frankenthal (DE); Wekel, Hans-Ulrich, Dr., 67158 Ellerstadt (DE)

(57) **Zusammenfassung**

Wässerige Zubereitungen, enthaltend ein Alkylpolyglykosid und ein Polyethylenglykoletherderivat der Formel I

R¹―O(̵CH₂-CH₂-O)̵ₙA(̵O-CH₂-CH₂)̵ₙO-R¹ I

worin
- R¹ =: Alkyl mit 8-22 Kohlenstoffatomen
- A =: von einem Diisocyanat abgeleiteter, 2-wertiger Rest und
- n =: 20 - 400
bedeutet.

## Beschreibung

Die vorliegende Erfindung betrifft wässerige Zubereitungen, die Alkylpolyglykoside und Polyethylenglykoletherderivate enthalten.

Bei den erfindungsgemäßen Zusammensetzungen handelt es sich insbesondere um kosmetische Mittel.

Aus dem Stand der Technik sind kosmetische Mittel bekannt, die Alkylpolyglykoside und Polymere enthalten (SÖFW-Journal, 121. Jahrgang, 8/95, Seiten 598-611). Es wird erwähnt, daß in Alkylpolyglykosid-Formulierungen Polymere wie z.B. Xanthan Gum und Alginate als Viskositäts-regelnde Zusatzmittel Verwendung finden (s.S. 607, 1. Sp.).

Aus der EP 511 566 A1 sind wässerige Tensidzubereitungen mit erhöhter Viskosität bekannt, die neben Basistensiden Alkylpolyglykoside und ein Polymer enthalten, wobei als Polymere Derivate alkoxylierter mehrwertiger Alkohole genannt sind.

Aus der EP 408 965 und der EP 388 810 sind "detergent compositions" bekannt, die Alkylglycoside enthalten.

Aus der DE 42 17 673 A1 sind elektrolytverdickbare Tensidkombinationen bekannt, die Alkylpolyglykoside, carboxymethyliertes Alkanoloxethylat und Monoalkylsulfosuccinat in ganz bestimmten Mengenverhältnissen enthalten.

Aus Colloids and Surfaces A: Physochemical and Engineering Aspects, 82 (1994) 263-277 sind hydrophobisch modifizierte Urethan-ethoxylat-Verdicker der Formel Alkyl-NHCO(̵Eₙ-DI)₂-Eₙ-OCONH-Alkyl mit E=Ethylenoxid und DI=Diisocyanat für den Einsatz in Anstrichmitteln bekannt.

In der DE 22 04 841 und der DE 20 54 885 sind Pigmentdruckpasten beschrieben, die als Verdickungsmittel Polyethylenglykoletherderivate enthalten.

Aufgabe der vorliegenden Erfindung war es, wässerige Zubereitungen zur Verfügung zu stellen, die Alkylpolyglykoside enthalten, d.h. ein Bestandteil auf Basis nachwachsender Rohstoffe, der aus toxikologischen, ökologischen und physiologischen Gesichtspunkten vorteilhaft ist. Gleichzeitig soll die Zubereitung, ohne daß größere Konzentrationen Elektrolyte (wie z.B. NaCl) erforderlich sind, eine hohe Viskosität aufweisen.

Diese Aufgabe wird durch wässerige Zubereitungen gelöst, die neben Alkylpolyglykosiden Polyethylenglykoletherderivate der Formel I enthalten

R¹―O(̵CH₂-CH₂-O)̵ₙA(̵O-CH₂-CH₂-CH₂)̵ₙO-R¹ I

worin
- R¹ =: Alkyl mit 8-22 Kohlenstoffatomen
- A =: von einem Diisocyanat abgeleiteter, 2-wertiger Rest und
- n =: 20 - 400
bedeutet.

Bei diesen Zubereitungen handelt es sich bevorzugt um kosmetische Mittel, insbesondere um Haarbehandlungsmittel, Duschbäder, Duschgele, aber auch Gesichtsreiniger, Handwaschpasten und Lotionen. Als Haarbehandlungsmittel sind zu nennen: Shampoos, Haarspülungen, Stylinggele und Wellmittel.

Die erfindungsgemäßen wässerigen Zubereitungen enthalten bevorzugt ein zusätzliches Tensid. Bei diesem Tensid kann es sich um in wässerigen, insbesondere kosmetischen Zubereitungen übliche Tenside handeln, wie z.B. Fettalkoholethersulfate, Fettalkoholsulfate, carboxymethylierte Fettalkoholoxethylate, Fettalkoholethersulfosuccinate, Alkansulfonate, fettsaure Salze, Alkylbetaine, Ampholyte, Fettalkoholoxethylate, Fettsäuresorbitanester, ethoxylierte Sorbitanester, Zuckerester sowie deren Gemische gelten als Basistenside, wobei die Kettenlänge der gesättigten oder ungesättigten, geradkettigen oder verzweigten Alkylkette jeweils 8 bis 22, vorzugsweise 10 bis 20 Kohlenstoffatome beträgt, und die Kationen der anionischen Tenside Na, K, NH₄, C₂-C₃-Alkanolammonium oder Mg sind. Die Ethoxylierungsgrade liegen bei den Fettalkoholethersulfaten zwischen 1 bis 5 (vorzugsweise zwischen 2 und 4), bei den carboxylierten Oxethylaten zwischen 2 und 15 (3 bis 10), bei den Fettalkoholethersulfosuccinaten zwischen 1 und 6 (2 bis 4), bei den Fettalkoholoxethylaten zwischen 2 und 25 (2 bis 15) mol Ethylenoxid/mol.

Die in den erfindungsgemäßen Zubereitungen enthaltenen Alkylpolyglykoside entsprechen bevorzugt der Formel II

R²-O-Gₘ II

worin
- R² =: einen linearen oder verzweigten Alkylrest mit 8 bis 18, bevorzugt 10-16 C-Atomen,
- G =: einen Polyglykosylrest und
- m =: 1,1 - 5, bevorzugt 1,1 - 2,5
bedeutet.

In obiger Formel I bedeuten R¹ bevorzugt einen Alkylrest mit 10-20 C-Atomen, A bevorzugt den zweiwertigen, von einem aromatischen Diisocyanat abgeleiteten Rest und n bevorzugt 60-300.

Die erfindungsgemäßen Zubereitungen enthalten vorzugsweise 1-20 Gew.-% Alkylpolyglykosid und 0,05-1, insbesondere 0,1-0,5 Gew.-% Polyethylenglykoletherderivate der Formel I.

Werden neben den Alkylpolyglykosiden und den üblichen Basistensiden die erfindungsgemäßen Polyethylenglykolderivate der Formel I eingesetzt, so erreicht man bereits mit Zusätzen von 0,2 bis 0,8 Gew.-% eine Verdickung von 4000-18000 mPa·s.

Die Herstellung der Polyethylenglykoletherderivate ist in der DE 22 04 841 im einzelnen angegeben, worauf ausdrücklich Bezug genommen wird.

Die erfindungsgemäß eingesetzten Alkylpolyglykoside sind kommerziell erhältlich, worauf im SÖFW-Journal, 121. Jahrgang, 8/95, Seite 598, hingewiesen wird. Sie sind auf bekannte Weise auf Basis nachwachsender Rohstoffe herstellbar, siehe z.B. EP 511 466 A1, Sp. 3. Dort wird angegeben, daß beispielsweise Dextrose in Gegenwart eines sauren Katalysators mit n-Butanol zu Butylpolyglykosidgemischen umgesetzt werden kann, welche mit langkettigen Alkoholen ebenfalls in Gegenwart eines sauren Katalysators zu den gewünschten Alkylpolyglykosidgemischen unglycosidiert werden.

Die Struktur der Produkte ist in bestimmten Grenzen variierbar. Der Alkylrest R² wird durch die Auswahl des langkettigen Alkohols festgelegt. Günstig aus wirtschaftlichen Gründen sind die großtechnisch zugänglichen Tensidalkohole mit 10 bis 18 C-Atomen, insbesondere native Fettalkohole aus der Hydrierung von Fettsäuren bzw. Fettsäurederivaten. Verwendbar sind auch Ziegleralkohol oder Oxoalkohole.

Der Polyglykosylrest Gₘ wird einerseits durch die Auswahl des Kohlenhydrats und andererseits durch die Einstellung des mittleren Polymerisationsgrades n z.B. nach DE-OS 19 43 689 festgelegt. Im Prinzip können bekanntlich Polysaccharide, z.B. Stärke, Maltodextrine, Dextrose, Galaktose,Mannose, Xylose etc. eingesetzt werden. Bevorzugt sind die großtechnisch verfügbaren Kohlenhydrate Stärke, Maltodextrine und besonders Dextrose. Da die wirtschaftlich interessanten Alkylpolyglykosidsynthesen nicht regio- und stereoselektiv verlaufen, sind die Alkylpolyglykoside stets Gemische von Oligomeren, die ihrerseits Gemische verschiedener isomerer Formen darstellen. Sie liegen nebeneinander mit α- und β-glykosidischen Bindungen in Pyranose- und Furanoseform. Auch die Verknüpfungsstellen zwischen zwei Saccharidresten sind unterschiedlich.

Erfindungsgemäß eingesetzte Alkylpolyglykoside lassen sich auch durch Abmischen von Alkylpolyglykosiden mit Alkylmonoglykosiden herstellen. Letztere kann man z.B. nach EP-A 0 092 355 mittels polarer Lösemittel, wie Aceton, aus Alkylpolyglykosiden gewinnen bzw. anreichern.

Der Glykosidierungsgrad wird zweckmäßigerweise mittels ¹H-NMR bestimmt.

Im Vergleich zu den in kosmetischen Reinigungsmitteln eingesetzten Tensiden gelten die Alkylpolyglykoside als überaus umweltverträglich. So liegt der mittels Kläranlagen-Simulationsmodell/DOC-Analyse bestimmte biologische Abbaugrad für die erfindungsgemäßen Alkylpolyglykoside bei 96 ± 3 %. Diese Zahl ist vor dem Hintergrund zu sehen, daß bei diesem Testverfahren (Totalabbau) bereits ein Abbaugrad >70 % bedeutet, daß die Substanz als gut abbaubar gilt.

Auch die akute orale Toxizität LD 50 (Ratte) sowie die aquatische Toxizität LC 50 (Goldorfe) und EC 50 (Daphnien) und Werten von >10000 mg/kg, 12 bzw. 30 mg/l liegen um den Faktor 3 bis 5 günstiger als die entsprechenden Werte der heute wichtigsten Tenside. Ähnliches gilt für die bei kosmetischen Formulierungen besonders wichtige Haut- und Schleimhautverträglichkeit.

Den erfindungsgemäßen Zubereitung können in geringen Mengen noch Salze wie z.B. Alkali-, Ammonium- oder/und Erdalkalihalogenide, -sulfate oder -phosphate zugesetzt werden.

Die erfindungsgemäßen Zubereitungen zeigen überraschenderweise auch dann eine gute Viskosität, wenn keine anionischen Tenside zugesetzt werden.

In bekannter Weise könne die erfindungsgemäßen wässerigen Zubereitungen weitere Komponenten enthalten, die für den jeweiligen Anwendungszweck bedeutsam sind. In Frage kommen Silikontenside, Eiweißhydrolysate, Duftstoffe, Trübungs- und Perlglanzmittel, Rückfetter, Silikonöle, Feuchthaltemittel, Konservierungsmittel, hautkosmetische Wirkstoffe, Pflanzenextrakte, Puffersubstanzen, Komplexbildner, etc.

### Beispiele

### Beispiel 1

### Verdickung einer Shampoogrundlage mit Natriumlaurylethersulfat (mit ca. 2 Mol EO, ca. 28 % Aktivsubstanz in H₂O) (Texapon® N 28) und Laurylpolyglycose (auf Basis C₁₂-C₁₆-Fettalkohol, mit einem Wert für m von 1,4-1,45) (Plantaren® 1200)

Rezeptur (Gew.-%):
22,0 Texapon® N 28
16,0 Plantaren® 1200
0,62 Citronensäure 10 %
0,5 Verdickungsmittel der Formel III
ad 100,0 Wasser

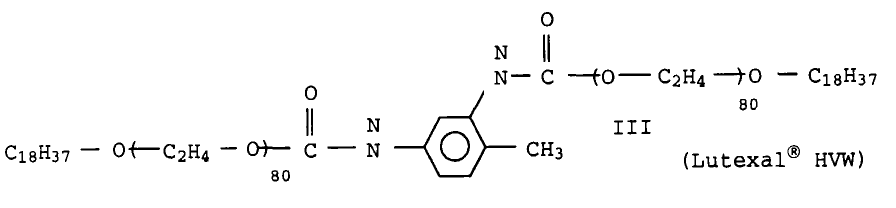

| Aussehen; | pH-Wert; | Viskosität (Haake VT 02, Spindel 2) |
|---|---|---|
| klar, dickflüssig; | pH: 6,06; | 1 500 mPa·s |

### Beispiel 2

### Verdickung einer Shampoogrundlage ohne Texapon® N 28

Rezeptur (Gew.-%):
30,0 Setacin 103 spezial (Disodium Laureth Sulfosuccinat)
18,0 Plantaren 1200
3,0 Amphotensid B 4 (Cocoamidopropyl Betain)
Verdickungsmittel wie unten angegeben
ad 100,0 Wasser

| Verd.mittel | Aussehen; | pH-Wert; | Viskosität (Haake VT 02; Spindel 1) |
|---|---|---|---|
| + 2 % Lutexal HVW | klar, dickflüssig | pH: 5,52 | 7000 mPa·s |
| + 2,0 % NaCl | klar, flüssig; | pH: 5,38 | 400 mPa·s (zu dünn) |

### Beispiel 3

### Shampoogrundlage verdickt mit Lutexal HVW - Konzentrationsreihe

Rezeptur (Gew.-%):
22,0 Texapon® N 28
16,0 Plantaren® 1200
0,62 Citronensäure 10 5
Verdickungsmittel wie unten angegeben
ad 100,0 Wasser

| Verd.mittel | Aussehen; pH-Wert; Viskosität (Haake VT 02; Spindel 1) | | |
|---|---|---|---|
| | nach Herstellung: | nach 24 h RT | nach 4 Tagen RT |
| + 0,35 % Lutexal HVW | klar, pH: 6,01, 1 400 mPa·s | klar, 1 700 mPa·s | klar, 1 800 mPa·s |
| + 0,40 % Lutexal HVW | klar, pH: 6,01, 2 100 mPa·s | klar, 2 900 mPa·s | klar, 2 800 mPa·s |
| + 0,50 % Lutexal HVW | klar, pH: 5,94, 4 200 mPa·s | klar, 5 000 mPa·s | klar, 5 000 mPa·s |
| + 0,60 % Lutexal HVW | klar, pH: 5,97, 8 500 mPa·s | klar, 9 500 mPa·s | klar, 9 500 mPa·s |

## Patentansprüche

1. Wässerige Zubereitungen, enthaltend ein Alkylpolyglykosid und ein Polyethylenglykoletherderivat der Formel I
R¹―O(̵CH₂-CH₂-O)̵ₙA(̵O-CH₂-CH₂)̵ₙO-R¹ I
worin
R¹ = Alkyl mit 8-22 Kohlenstoffatomen
A = von einem Diisocyanat abgeleiteter, 2-wertiger Rest und
n = 20 - 400
bedeutet.

2. Zubereitungen nach Anspruch 1, dadurch gekennzeichnet, daß es sich bei den Zubereitungen um kosmetische Mittel handelt.

3. Zubereitungen nach Anspruch 2, dadurch gekennzeichnet, daß die kosmetischen Mittel Haarbehandlungsmittel, Duschbäder oder Duschgele sind.

4. Zubereitungen nach Anspruch 1, dadurch gekennzeichnet, daß sie zusätzlich ein Tensid enthalten.

5. Zubereitungen nach Anspruch 1, dadurch gekennzeichnet, daß sie Alkylpolyglykoside der folgenden Formel II enthalten
R²-O-Gₘ II
worin
R² = einen linearen oder verzweigten Alkylrest mit 8 bis 18 C-Atomen oder Gemischen davon,
G = einen Polyglykosylrest und
m = 1,1 - 5
bedeutet.

6. Zubereitung nach Anspruch 1, dadurch gekennzeichnet, daß in Formel I
R¹ = Alkyl mit 10-20 C-Atomen
A = einen von einem aromatischen Diisocyanat abgeleiteten, 2-wertigen Rest und
n = 60 - 300
bedeutet.

7. Zubereitung nach Anspruch 5, dadurch gekennzeichnet, daß in Formel II
R² = einen linearen Alkylrest mit 10-16 C-Atomen und
n = 1,1 bis 2,5
bedeutet.

8. Zubereitungen nach Anspruch 1, dadurch gekennzeichnet, daß sie
1 bis 20 Gew.-% Alkylpolyglykosid und
0,05 bis 1 Gew.-% Polyethylenglykoletherderivat der Formel I
enthalten.
